Europäisches Patentamt

European Patent Office

Office européen des brevets

(11) Numéro de publication: **0 311 532 B1**

(12) **FASCICULE DE BREVET EUROPEEN**

(45) Date de publication de fascicule du brevet: **10.08.94**  (51) Int. Cl.⁵: **A61B 19/00**

(21) Numéro de dépôt: **88420330.8**

(22) Date de dépôt: **03.10.88**

(54) **Housse de protection.**

(30) Priorité: **07.10.87 FR 8714076**

(43) Date de publication de la demande:
**12.04.89 Bulletin 89/15**

(45) Mention de la délivrance du brevet:
**10.08.94 Bulletin 94/32**

(84) Etats contractants désignés:
**AT BE CH DE ES GB GR IT LI LU NL SE**

(56) Documents cités:
**WO-A-86/06641**
**FR-A- 2 529 788**
**FR-A- 2 578 746**
**GB-A- 2 150 028**

(73) Titulaire: **Grivon, Josette**
**8 rue Jean Mermoz**
**F-74300 Cluses (FR)**

(72) Inventeur: **Grivon, Josette**
**8 rue Jean Mermoz**
**F-74300 Cluses (FR)**

Rank Xerox (UK) Business Services
(3.10/3.09/3.3.3)

## Description

La présente invention concerne des housses de protection pour éviter la prolifération des microbes,virus,bactéries,dans les différents appareils ou instruments de régulation ou autres utilisés à usages médicaux.

Les appareils de régulation(rampes 2,3,4,5 robinets,fluch poussoir,robinets à 3 voies, tous raccords ou bouchons)peuvent être un risque de développement des microbes.Ces housses sont utilisées pour isoler les appareils qui servent à la régulation des sérums,plasmas ou autres liquides venant de bocaux ou machines et alimentant ou éliminant les liquides allant ou venant du malade.D'autres housses sont utilisées pour les connecteurs ou bouchons servant périodiquement mais restant toujours fixés sur le malade; le principe de la housse est le même,ce sont les dimensions qui changent. Ces housses restent et protègent les appareils en cours de fonctionnement, ce qui permet de déplacer sans risque le malade,tout le matériel étant bien à l'intérieur de la housse dans son cocon de désinfectant actif.

Il existe des boitiers en matière rigide tel que celui décrit dans le Brevet FR 2 578 746.Un tel boitier en matière rigide force la mousse des deux parties du dit boitier à entrer en contact et à protéger ainsi l'appareillage à usage médical ,des bactéries ou des microbes.

Il est apparu de façon surprenante qu'une housse de protection appelée également protecteur réalisé en matériau souple selon l'invention permette de maintenir en condition aseptique satisfaisante les appareillages de prélévements ou d'injection à usage médical.

De plus,de part sa conception et sa réalisation en matériaux souples,la housse ou protecteur apporte des avantages non négligeables:confort du malade,la housse peut glisser involontairement sous le corps d'un malade sans meurtrissure pour celui-ci de par l'épaisseur de la mousse qui enrobe parfaitement le profil du ou des appareils à protéger; grande maniabilité des appareils sans la complète ouverture de la housse(l'opérateur peut d'une main maintenir housse et appareil et de l'autre main entr'ouvrir la housse et actionner les robinets ou seringues.)

Le boitier rigide connu comprend,sur ses faces latérales,des ouvertures permettant le passage des tubulures dans lesquelles il faut impérativement placer celles-ci si on ne veut pas risquer un pincement génant voir dangereux pour le malade.

Sur la housse de protection ou protecteur, on peut faire sortir la ou les tubulures en n'importe quel point des trois faces de fermeture auto-agrippantes.De plus, le nombre de sorties n'est pas impérativement limité.

L'invention concerne donc une housse de protection pour divers appareils ou instruments de régulation à usages médicaux , caractérisée en ce qu'elle comporte un support d'un film d'un matériau souple , une couche de mousse dont la surface est plus petite que celle du support, centrée et fixée sur le support , des moyens latéraux de fermetures auto-agrippantes à crochets et boucles

pour fermer la housse sur elle-même en y maintenant intérieurement un appareil, après l'avoir repliée suivant son axe médian (A, A').

L'invention concerne egalement une housse de protection pour divers appareils ou instruments de régulation à usages médicaux , caractérisée en ce qu'elle comporte deux supports indépendants assemblés l'un à l'autre , chaque support étant en un film d'un matériau souple, une couche de mousse dont la surface est plus petite que celle du support, centrée et fixée sur le support , des moyens latéraux de fermetures auto-agrippantes à crochets et boucles

pour fermer la housse en y maintenant intérieurement un appareil.

La housse de protection est composée d'un film souple de préférence en polyéthylène,d'une mousse de polyuréthane, de bandes auto-agrippantes.

Le film est en polyéthylène ou dérivés ayant les mêmes propriétés physiques et chimiques;son épaisseur de l00 à l50 microns permet d'obtenir une élasticité et une souplesse d' utilisation tout en restant suffisament résistante.Les dimensions (longueur et largeur)sont définies en fonction des appareils à conditionner à l'intérieur,la gamme étant très étendue mais le principe de fabrication de la housse ne change pas.Le film est en un matériau ayant les propriétés,de souplesse,élasticité,imperméabilité.

On peut aisément,maintenir l'appareil,ou l'instrument pendant les manipulations des robinets grâce à la souplesse du film sans risque de contamination;les manipulations sont très fréquentes en cours de journée,une opération toutes les deux heures environ.

L'élasticité de ce film permet de protéger tout appareil des chocs et de la pénétration des microbes,bactéries,virus, en les enveloppant comme un gant fourré.

L'imperméabilité du fait de sa matière plastique son imperméabilité est assurée;les produits actifs mis à l'intérieur pour aseptiser les instruments ou autres appareils ne peuvent s'échapper,ainsi que les souillures ou autres corps étrangers de l'extérieur ne peuvent pénétrer ce qui assure cette sécurité essentielle dans les centres hospitaliers,qui sont des milieux de germes microbiens.

La mousse est de préférence en polyuréthane,sa densité est de l6 à 2l Kgs/m3,son épaisseur de l0mm environ de façon à bien recouvrir l'objet et que l'élasticité soit suffisante pour que la surface de mousse en regard enrobe l'objet dans son intégralité.Cette mousse a un pouvoir d'absorption très grand et reste après 17 heures encore imprégnée comme les tests l'ont montré,si bien que les housses peuvent n'être changées que tous les 3 ou 4 jours suivant les services.Cette mousse est formée de milliers d'alvéoles dont certaines sont ouvertes,d'autres fermées(environ l fermée pour 4 ouvertes)ce qui permet une pénétration rapide des produits actifs tout en maintenant ce produit pendant la durée de l'utilisation de la housse.Afin de maintenir constamment l'efficacité de l'aseptie des appareils,on peut,par mesure de sécurité,réimbiber la mousse de petites doses 2 fois par 24 h. La mousse est fixée sur un support formé d'une trame enduite de colle chauffée ce qui permet une très bonne adhérence sur le film,même en présence d'un produit actif qui,après quelques heures,devient collant:cela ne pose aucun problème pour la housse.Ce produit actif et très efficace commercialisé sous le nom de Bétadine est le plus utilisé dans les milieux médicaux.

Cette mousse est posée sur le film de préférence de façon équidistante sur 3 côtés à 25mm environ des bords du film polyéthylène,le 4ème côté dans le sens de la longueur à 40mm pour laisser une partie du film en forme de languette afin de permettre une meilleure prise en main,d'où une certaine facilité d'ouverture et rapidité très appréciées du personnel hospitalier.

Le système de fermeture se compose de bandes de l0mm de large qui permettent une fermeture hermétique, tout en laissant à n'importe quel point, le passage des tubulures distribuant les différents liquides des bocaux vers le malade.Ces bandes sont formées d'un tissus adhésivé,un côté boucles,un côté crochets, commercialisées sous la marque velcro.La bande crochets étant mis en contact avec la bande boucles,on obtient une fermeture agrippante hermétique.Afin de renforcer l'adhérence des parties auto-agrippantes sur le film de polyéthylène celles-ci sont thermo-soudées ce qui permet de renforcer également l'étanchéité de la pochette ainsi formée, une fois replié.Entre la mousse et les bandes auto-agrippantes,un espace de 5mm environ est laissé afin de permettre le gonflement de la mousse lors de l' imprégnation du produit actif,de même qu'en présence des divers appareils à l'intérieur de la housse.

La housse ou protecteur possède des dimensions adaptées aux instruments ou appareils de façon à ce qu'ils soient entièrement enrobés par la mousse;par exemple, pour une rampe de 3 ou 4 robinets, on peut adapter une seringue pour des injections periodiques pour un produit s'injectant toutes les 2 h. (héparine ou insuline):la seringue est alors laissée à l'intérieur de la housse à l'abri de tous les microbes;la housse de ce fait a des dimensions appropriées.

La housse se compose d'un support repliable(l) sur luimême,au centre la mousse(2),les fermetures auto-agrippantes les unes en face des autres ou bien en 2 supports indépendants assemblés l'un à l'autre.

Les dessins annexés illustrent l'invention,les figures de l à 4 montrent des exemples de réalisation de l'invention

La figure l représente le film polyéthylène ou dérivés mais possédant les mêmes propriétés.

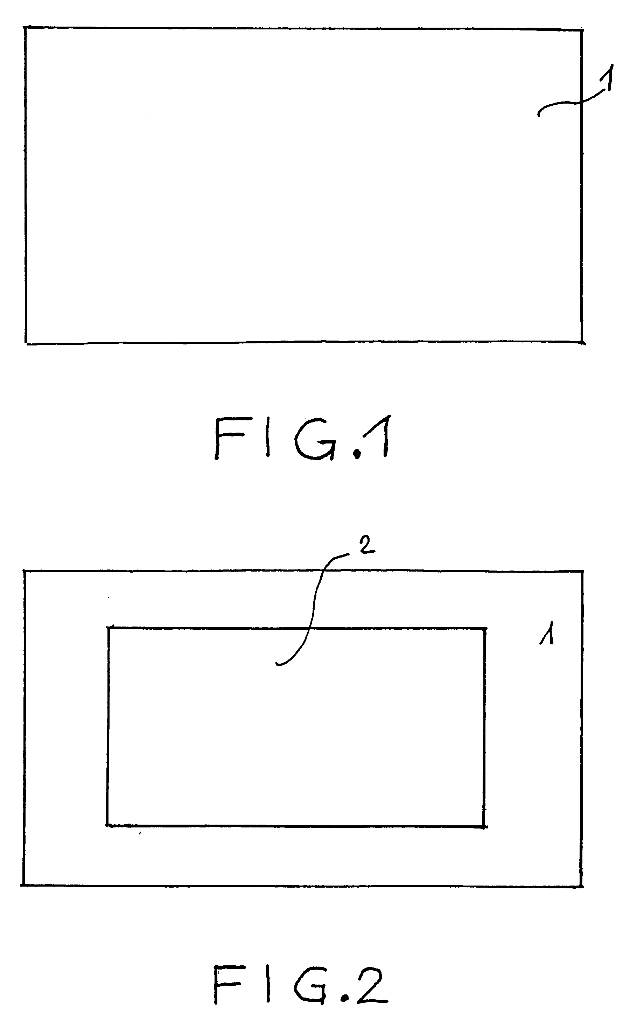

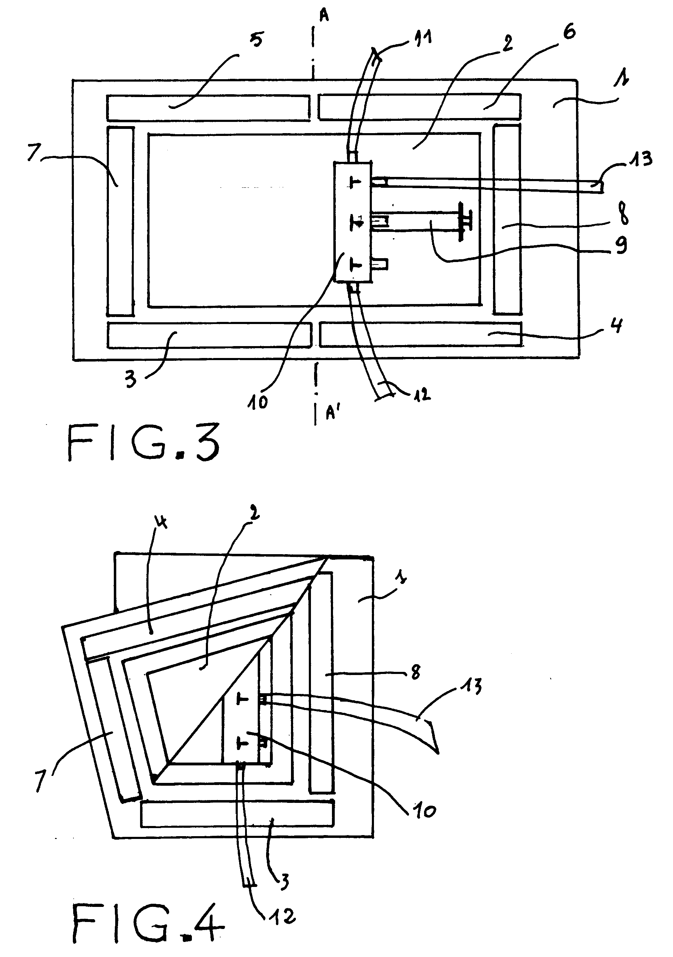

La figure 2 représente le film de polyéthylène(l) avec, collée, la plaque de mousse(2) en polyuréthane adhésivée.

La figure 3 représente le film de polyéthylène (l), plus la plaque de mousse (2), plus les bandes auto-agrippantes adhévées thermo-soudées (3,5,7) crochets,(4,6,8) boucles,à l'intérieur une rampe à 3 robinets (l0) sur laquelle est fixée en permanence une seringue (9) et deux tubulures (ll,l2) amenant le sérum ou autres liquides des bocaux à l'appareil,la tubulure (l3) distribuant ces liquides au malade.

La figure 4 représente une housse ou protecteur ouvert sur deux côtés laissant apparaitre le film (l) ,la plaque de mousse (2), les bandes auto-agrippantes (3,7), crochets (4,8) boucles, rampe à 3 robinets (l0), les tubulures (l2,l3).

La housse et ses constituants résistent à toutes sortes de stérilisation(formol ,oxyde d'ethylène ...)

Il est possible d'adapter un système de fixation de la housse sur le lit ou sur le bras du malade ou sur le fauteuil roulant dans le cas de déplacement du patient; une pastille adhésivée sera fixée sur la housse ,une autre sera fixée sur le support choisi,ceci à la demande de l'utilisateur.

Des études ont été effectuées pour prouver l'efficacité d' aseptie bactériologique d'un protecteur de rampe à 4 robinets selon la série I voir tableau I de tests; le protecteur était constitué d'un champ de tissus stérile utilisé couramment en milieu hospitalier.Dans les tests de la série 2 voir tableauII un protecteur de rampe à 4 robinets était utilisé.

Selon la série I, voir tableau III, le nombre de colonies de bactéries par rampe était de l2l,40 alors que dans la série2 voir tableau III avec un protecteur de rampe selon l'invention il n'est que de II,89.

D'autres tests montrant la facilité de manipulation du protecteur de rampe selon l'invention série 2 voir tableau IV comparatif par rapport au champ de tissus stérile série I, voir tableau IV; en effet lorsque cette rampe est confinée dans un champ de tissus stérile, l'antiseptique se dessèche plus rapidement que dans

EP 0 311 532 B1

la série 2 où le protecteur souple selon l'invention est utilisé.Ce déssèchement prématuré de l'antiseptique série I oblige à des manipulations plus fréquentes des robinets de la rampe.

Les tableaux 1 et II montrent l'efficacité comparative du protecteur selon l'invention contre la contamination bactériologique avec un champ stérile.

Tableau III

| Résultats statistiques | | | | |
|---|---|---|---|---|
| | Nombre de rampes | Moyenne du nombre de colonies par rampe | Ecart-type | Test de Mann- Whitney |
| Série 1 | 20 | 121,40 | 69,97 | $p < 0,05$ |
| Sériè 2 | 19 | 11,89 | 19,55 | |

Tableau IV

| Tableau comparatif du nombre de manipulations entre les 2 séries | | |
|---|---|---|
| | Moyenne des ouvertures des protecteurs par 24 h | Moyenne des manipulations des robinets par 24 h |
| Série 1 | 5,5 | 3,6 |
| Série 2 | 4,45 | 2,9 |

4

Tableau I

Contamination bactériologique des rampes à 4 robinets protégées pendant 24 h par un champ tissu stérile

SERIE I

| Série 1 n° rampe prélevée | Nombre de colonies décrochées | | | | Identification | Malade connu infecté à Staphylococcus aureus | Durée de séjour en jours | Nb. moyen ouvertures champ par 24 h | Nb. moyen manipulations robinets par 24 h | Nb. moyen manipulations connections par 24 h |
|---|---|---|---|---|---|---|---|---|---|---|
| | décr. 1 | décr. 2 | décr. 3 | total | | | | | | |
| 1 | 40 | 3 | 0 | 43 | Staphylococcus epdermidis | | 6 | 9 | 6 | 3 |
| 2 | 100 | 2 | 0 | 102 | Staphylococcus species Micrococcus Bacillus | | 6 | 4 | 4 | 4 |
| 3 | 100 | 100 | 60 | 260 | Micrococcus | CBT | 9 | 3 | 2 | 2 |
| 4 | 110 | 21 | 4 | 135 | Non identifiées | CBT | 26 | 4 | 2 | 2 |
| 5 | 150 | 14 | 5 | 169 | Non identifiées | | 7 | 6 | 5 | 3 |
| 6 | 100 | 40 | 11 | 151 | Staphylococcus epdermidis Bacillus Sarcines | | 17 | 3 | 2 | 2 |
| 7 | 77 | 4 | 2 | 83 | Staphylococcus epdermidis Sarcine | | 33 | 7 | 6 | 2 |
| 8 | 150 | 30 | 20 | 200 | Staphylococcus epdermidis Bacillus | | 27 | 5 | 3 | 2 |

EP 0 311 532 B1

EP 0 311 532 B1

| Série 1 n° rampe prélevée | Nombre de colonies décrochées | | | | Identification | Malade connu infecté à Staphylococcus aureus | Durée de séjour en jours | Nb. moyen ouvertures champ par 24 h | Nb. moyen manipulations robinets par 24 h | Nb. moyen manipulations connections par 24 h |
|---|---|---|---|---|---|---|---|---|---|---|
| | décr. 1 | décr. 2 | décr. 3 | total | | | | | | |
| 9 | 150 | 8 | 2 | 160 | Staphylococcus epdermidis Micrococcus | | 6 | 11 | 4 | 4 |
| 10 | 200 | 20 | 7 | 227 | Staphylococcus epdermidis Bacillus | | 30 | 4 | 3 | 2 |
| 11 | 100 | 8 | 3 | 111 | Staphylccoccus epdermidis 7 | | non déterminée | 5 | 2 | 2 |
| 12 | 17 | 2 | 0 | 19 | Staphylococcus aureus Staphylococcus epdermidis 50 | | 30 | 4 | 3 | 3 |
| 13 | 150 | 4 | 2 | 156 | Staphylococcus aureus Staphylococcus epdermidis | | non déterminée | 4 | 4 | 2 |
| 14 | 17 | 0 | 12 | 29 | Staphylococcus epdermidis | | 22 | 2 | 2 | 2 |

| Série 1 n° rampe prélevée | Nombre de colonies décrochées | | | | Identification | Malade connu infecté à Staphylococcus aureus | Durée de séjour en jours | Nb. moyen ouvertures champ par 24 h | Nb. moyen manipulations robinets par 24 h | Nb. moyen manipulations connections par 24 h |
|---|---|---|---|---|---|---|---|---|---|---|
| | décr. 1 | décr. 2 | décr. 3 | total | | | | | | |
| 15 | 100 | 4 | 3 | 107 | Staphylococcus epdermidis Bacillus Sarcine | Broche osseuse | 41 | 5 | 2 | 2 |
| 16 | 100 | 1 | 0 | 101 | Staphylococcus epdermidis Sarcine | | 42 | 8 | 4 | 4 |
| 17 | 150 | 30 | 12 | 192 | Staphylococcus epdermidis Sarcine | Drain thoracique | 13 | 9 | 7 | 4 |
| 18 | 100 | 20 | 8 | 128 | Staphylococcus epdermidis Sarcine | | 10 | 4 | 2 | 2 |
| 19 | 30 | 2 | 1 | 33 | Staphylococcus aureus Staphylococcus epdermidis Corynebacterium | | 3 | 6 | 4 | 2 |
| 20 | 18 | 0 | 4 | 22 | Staphylococcus epdermidis | | non déterminée | 7 | 5 | 3 |

Tableau II

Contamination bactériologique des rampes à 4 robinets protégées pendant 96 h par un protecteur mousse stérile puis bétadiné

SERIE 2

| Série 2 n° rampe prélevée | Nombre de colonies décrochées | | | | Identifica-tion | Malade connu infecté à Staphylo-coccus aureus | Durée de séjour en jours | Nb. moyen ouvertures protecteur par 24 h | Nb. moyen manipula-tions robinets par 24 h | Nb. moyen manipula-tions connec-tions par 24 h | Nb moyen applications Bétadine par 24 h |
|---|---|---|---|---|---|---|---|---|---|---|---|
| | décr 1 | décr 2 | décr 3 | total | | | | | | | |
| 1 | 8 | 1 | 4 | 13 | Staphylo-coccus epidermidis Bacillus Sarcine | | 4 | 5 | 4 | 2 | 2 |
| 2 | 2 | 9 | 2 | 13 | Staphylo-coccus epidermidis Bacillus Sarcine | | 32 | 5 | 4 | 3 | 3 |
| 3 | 11 | 10 | 1 | 22 | Staphylo-coccus epidermidis Bacillus *Levures* | | 8 | 4 | 2 | 2 | 2 |
| 4 | 5 | 1 | 3 | 9 | Staphylo-coccus epidermidis Bacillus | | 19 | 5 | 5 | 3 | 1 |
| 5 | 0 | 0 | 2 | 2 | Bacillus | | 6 | 4 | 2 | 2 | 2 |
| 6 | 3 | 2 | 0 | 5 | *non identifiées* | Redon | 10 | 5 | 4 | 2 | *non déterminé* |

EP 0 311 532 B1

8

| Série 2 n° rampe prélevée | Nombre de colonies décrochées | | | | Identifica-tion | Malade connu infecté à Staphylo-coccus aureus | Durée de séjour en jours | Nb. moyen ouvertures protecteur par 24 h | Nb. moyen manipula-tions robinets par 24 h | Nb. moyen manipula-tions connec-tions par 24 h | Nb moyen applications Bétadine par 24 h |
|---|---|---|---|---|---|---|---|---|---|---|---|
| | décr 1 | décr 2 | décr 3 | total | | | | | | | |
| 7 | 50 | 20 | 7 | 77 | Staphylo-coccus epidermidis Bacillus Micrococ-cus | | 13 | 3 | 3 | 3 | non déterminé |
| 8 | 0 | 0 | 6 | 6 | Staphylo-coccus epidermidis | | 33 | 4 | 2 | 2 | 2 |
| 9 | 2 | 2 | 1 | 5 | Staphylo-coccus epidermidis Corynebac-terium | | 12 | 3 | 1 | 1 | 2 |
| 10 | 0 | 1 | 0 | 1 | Bacillus | | 34 | 4 | 2 | 2 | 2 |
| 11 | 10 | 0 | 1 | 11 | Staphylo-coccus epidermidis Bacillus | | 6 | 5 | 3 | 3 | 2 |
| 12 | 1 | 0 | 0 | 1 | Staphylo-coccus epidermidis | Drain thoracique | 19 | 4 | 2 | 1 | 2 |
| 13 | 1 | 0 | 0 | 1 | Staphylo-coccus epidermidis | | 55 | 4 | 2 | 1 | 2 |
| 14 | 1 | 0 | 0 | 1 | Staphylo-coccus epidermidis | | 19 | 5 | 3 | 2 | 2 |

| Série 2 n° rampe prélevée | Nombre de colonies décrochées | | | | Identification | Malade connu infecté à Staphylococcus aureus | Durée de séjour en jours | Nb. moyen ouvertures protecteur par 24 h | Nb. moyen manipulations robinets par 24 h | Nb. moyen manipulations connections par 24 h | Nb. moyen applications Bétadine par 24 h |
|---|---|---|---|---|---|---|---|---|---|---|---|
| | décr 1 | décr 2 | décr 3 | total | | | | | | | |
| 15 | 30 | 20 | 0 | 50 | Staphylococcus epidermidis Bacillus | | 23 | 4 | 3 | 2 | 3 |
| 16 | 2 | 1 | 0 | 3 | Staphylococcus epidermidis | CBT | non déterminée | 4 | 2 | 2 | 2 |
| 17 | | 1 | 0 | 1 | non identifiées | | 7 | 4 | 2 | 2 | 3 |
| 18 | | 0 | 0 | 3 | Staphylococcus epidermidis Bacillus Sarcine | Plaie | 12 | 4 | 2 | 1 | 1 |
| 19 | 0 | 2 | 0 | 2 | non identifiées | | 9 | 9 | 8 | 2 | 2 |

## Revendications

1. Housse de protection pour divers appareils ou instruments de régulation à usages médicaux , caractérisée en ce qu'elle comporte un support d'un film (1) d'un matériau souple , une couche de mousse (2) dont la surface est plus petite que celle du support, centrée et fixée sur le support (1), des moyens latéraux de fermetures auto-agrippantes à crochets (3, 5, 7) et boucles ( 4, 6, 8 ) pour fermer la housse sur elle-même en y maintenant intérieurement un appareil, après l'avoir repliée suivant son axe médian (A, A').

2. Housse de protection pour divers appareils ou instruments de régulation à usages médicaux , caractérisée en ce qu'elle comporte deux supports indépendants assemblés l'un à l'autre , chaque support étant en un film (1) d'un matériau souple, une couche de mousse (2) dont la surface est plus petite que celle du support, centrée et fixée sur le support (1), des moyens latéraux de fermetures auto-agrippantes à crochets (3, 5,7) et boucles (4, 6, 8) pour fermer la housse en y maintenant intérieurement un appareil.

3. Housse selon l'une des revendications 1 ou 2 caractérisée en ce que le film (1) a une épaisseur de 100 à 150 microns et est constitué d'un matériau de type polyéthylène ou dérivés.

4. Housse selon l'une des revendications 1 à 3 caractérisée en ce que la couche de mousse (2) a une épaisseur de 10mm et est constituée d'un matériau absorbant de type polyuréthane de densité 16 à 21kg/m3, présentant une proportion majeure d'alvéoles ouvertes et des alvéoles fermées.

5. Housse selon l'une des revendications 1 à 4 , caractérisée en ce que la couche de mousse (2) est positionnée sur le support (1) de manière à réserver sur deux côtés latéraux opposés et sur l'un des côtés transversaux une bande d'une longueur de 25mm et sur l'autre côté transversal une bande d'une longueur de 40mm de manière à ménager dans le film support une languette de manipulation.

6. Housse selon l'une des revendications 1 à 5 , caractérisée en ce que la couche de mousse (2) est fixée sur une trame enduite de colle thermoadhérente pour sa fixation sur le support (1).

7. Housse selon l'une quelconque des revendications précédentes, caractérisée en ce qu'elle est constituée de matériaux pouvant être stérilisés.

8. Housse selon l'une quelconque des revendications précédentes, caractérisée en ce que le film (1) est en un matériau élastique.

9. Utilisation de la housse selon les revendications 1 à 8 pour la protection d'appareils ou instruments pourvus de robinets (10).

10. Utilisation de la housse selon les revendications 1 à 8 pour la protection d'appareils ou instruments pourvus de tubulures (11, 12, 13) amenant des liquides ou les distribuant.

**Claims**

1. Protective cover for various regulation equipment or instruments used for medical purposes , characterized in that it includes a support made of a film (1)of supple material , a layer of foam (2), the area of which is smaller than that of the support and that is centred and attached to the support (1), of lateral means of self-gripping closing systems with hooks (3,5,7) and buckles (4,6,8) that close the cover onto itself while holding an instrument inside after , after having folded it along its median axis (A,A') .

2. Protective cover for various regulation equipment or instruments used for medical purposes , characterized in that it includes two independent supports joined one with the other , each support being made of a film (1) of supple material , a layer of foam (2), the area of which is smaller than that of the support and that is centred and attached to the support (1) , of lateral means of self-gripping closing systems with hooks (3,5,7) and buckles (4,6,8) that close the cover onto itself while holding an instrument inside .

3. Cover according to one of claims 1 or 2 , characterized in that the film (1) has a thickness of 100 to 150 microns and is made of a material like polyethylene or derivative .

4. Cover according to one of claims 1 to 3 , characterized in that the layer of foam (2) has a thickness of 10 mm and is made of an absorbant material like polyurethane with a density of 16 to 21 kg/m3 containing a high proportion of open alveoli and some closed ones .

5. Cover according to one of claims 1 to 4 , characterized in that the layer of foam (2) is placed on the support (1) so as to leave aside a 25 mm long strip on two lateral and opposite sides and on one of the transverse sides , a 40 mm long strip on the other transverse side so as to keep a tongue-shaped piece in the support film for manipulation purposes .

6. Cover according to one of claims 1 to 5 , characterized in that the layer of foam (2) is attached to a framework coated with thermo-adhesive glue for attaching it to the support (1) .

7. Cover according to any of preceding claims , characterized in that it is made of materials that can be sterilized.

8. Cover according to any of preceding claims , characterized in that the film (1) is made of elastic material.

9. Usage of the cover according to claims 1 to 8 for the protection of equipment or instruments having taps (10).

10. Usage of the cover according to claims 1 to 8 for the protection of equipment or instruments having tubulures (11,12,13) that bring in or distribute liquids.

**Patentansprüche**

1. Schutzüberzug für verschiedene medizinische Regelapparate oder -instrumente, dadurch gekennzeichnet, daß er umfaßt einen Trägerfilm (1) aus einem weichen Material, eine Schaumstoffschicht (2), die kleiner als der Träger ist und auf diesem Träger (1) zentral fixiert ist, und laterale selbsthaftende Verschlüsse mit Haken (3, 5, 7) und Ösen (4, 6, 8), um den Überzug nach Faltung um seine transversale Mittelachse (A A') unter Einschluß eines Apparates zu verschließen.

2. Schutzüberzug für verschiedene medizinische Regelapparate oder -instrumente, dadurch gekennzeichnet, daß er zwei unabhängige zusammengefügte Träger umfaßt, wobei jeder Träger besteht aus einem Film (1) aus einem weichen Material, aus einer Schaumstoffschicht (2), die kleiner als der Träger ist und auf diesem Träger (1) zentral fixiert ist, und aus lateralen selbsthaftenden Verschlüssen mit Haken (3, 5, 7) und Ösen (4, 6, 8), um den Überzug unter Einschluß eines Apparates zu verschließen.

3. Überzug gemäß einem der Ansprüche 1 oder 2, dadurch gekennzeichnet, daß der Film (1) zwischen 100 und 150 Mikrons dick ist und aus einem Material vom Polyethylentyp oder dessen Derivaten besteht.

4. Überzug gemäß einem der Ansprüche 1 bis 3, dadurch gekennzeichnet, daß die Schaumstoffschicht (2) 10 mm dick ist, daß sie aus einem saugfähigen Material vom Polyurethantyp besteht, das eine Dicke von 16 bis 21 kg/m$^3$ und mehr offene als geschlossene Alveolen hat.

5. Überzug gemäß einem der Ansprüche 1 bis 4, dadurch gekennzeichnet, daß die Schaumstoffschicht (2) so auf dem Träger (1) aufgebracht ist, daß der Rand an zwei gegenüberliegenden lateralen Seiten sowie an einer transversalen Seite 25 mm und an der anderen transversalen Seite 40 mm lang ist, so daß auf dem Trägerfilm ein Streifen zur Handhabung bleibt.

6. Überzug gemäß einem der Ansprüche 1 bis 5, dadurch gekennzeichnet, daß die Schaumstoffschicht (2) zur Befestigung auf dem Träger (1) auf ein mit Thermokleber beschichtetes Gewebe aufgebracht ist.

7. Überzug gemäß einem der obigen Ansprüche, dadurch gekennzeichnet, daß er aus sterilisierbaren Materialien besteht.

**8.** Überzug gemäß einem der obigen Ansprüche, dadurch gekennzeichnet, daß der Film (1) aus einem elastischen Material besteht.

**9.** Verwendung des Überzugs gemäß den Ansprüchen 1 - 8 zum Schutz von Geräten oder Instrumenten, die mit Hähnen (10) versehen sind.

**10.** Verwendung des Überzugs gemäß den Ansprüchen 1 - 8 zum Schutz von Geräten oder Instrumenten, die mit flüssigkeitsführenden oder -verteilenden Leitungen (11, 12, 13) versehen sind.

FIG.1

FIG.2

FIG.3

FIG.4